# EUROPEAN PATENT APPLICATION

(11) **EP 1 035 202 A2**
(43) Date of publication of application: **13.09.2000**
(21) Application number: 00104485.8
(22) Date of filing: 09.03.2000
(51) Int. Cl.: C12N 15/10, C12N 15/66, C12N 15/31, C12N 1/21, C07K 14/315, C07K 16/12, C12Q 1/68

(54) **A cloning method for DNA fragments using arbitrarily primed PCR**

(30) Priority: 11.03.1999 JP 6443299
(71) Applicant: JCR PHARMACEUTICALS CO., LTD., Ashiya, Hyogo 659-0021 (JP)
(72) Inventor: Hong, Kyongsu, Nada-ku, Kobe-shi, Hyogo 657-0825 (JP)
(74) Representative: Reinhard - Skuhra - Weise & Partner

(57) **Abstract**

A method of cloning DNA fragments is disclosed comprising the steps of: (a) cleaving a vector DNA of plasmid origin with a restriction enzyme, (b) dephosphorylating an end of thus obtained cleaved DNAs, (c) separately obtaining a mixture of DNA fragments by cleaving a chromosomal DNA of a given organism with the same restriction enzyme as the aforementioned restriction enzyme, (d) obtaining a mixture of ligated DNAs through ligation using the dephosphorylated DNAs obtained in step (b) and the mixture of DNA fragments obtained in step (c), (e) amplifying the ligated DNAs by PCR with arbitrary primers using the obtained mixture of ligated DNAs as templates, and (f) introducing into a competent cell a cloning vector into which is incorporated a PCR product thus obtained.

## Description

### FIELD OF THE INVENTION

The present invention relates to a method of cloning fragments of an unknown gene using arbitrary primers, as well as to nucleotide and amino acid sequences of a gene which had been unknown and was cloned by the method.

### BACKGROUND OF THE INVENTION

There have been known methods of cloning genes. In general, cloning of a gene has been performed based upon the inherent functions of the protein coded by the gene or based upon a nucleotide sequences estimated from a fragment of that protein. In recent years, after the development of PCR (polymerase chain reaction), it has become possible to carry out amplification using oligonucleotides consisting of several tens of nucleotides at 5'- and 3'-ends of a known gene, respectively, without relying on the functions the protein, and then, after purification of the amplification product, cloning using a cloning vector. This method, however, still requires that the sequences of several tens of nucleotides at 5'- and 3'-ends are known, respectively. There was developed an improved method, SSP-PCR (single specific primer-PCR). This is a method for cloning a gene, known or unknown, in which a known sequence of several tens of nucleotides at its 5'- or 3'-end and a arbitrary primer are utilized. There is known, however, no method of directly cloning fragments of an unknown gene utilizing arbitrary primers only.

Thus, the conventional PCR requires information about several tens of nucleotides at 5'- and 3'-ends, and the improved method, SSP-PCR requires information about a nucleotide sequence made of several tens of nucleotides at 5'- or 3'-end. Therefore, an established method using arbitrary primers for directly cloning a fragment of a gene whose nucleotide sequence is not known would enable to preferentially select unknown genes, thereby allowing to efficiently finding new genes which might have potential applications such as therapeutics, for example. Thus, the objective of the present invention is to provide a method which enables cloning unknown genes using arbitrary primers.

### SUMMARY OF THE INVENTION

Through investigations from the above-mentioned viewpoint, the present inventor found that, by dephosphorylating an end of a linear DNA obtained by cleaving a vector of plasmid origin, e.g., a vector derived from pUC, with a restriction enzyme, then ligating the cleaved product with a mixture of fragments of a chromosomal DNA obtained by digestion of the DNA carrying the aimed unknown gene with the same restriction enzyme, amplifying the ligation products by PCR using arbitrary primers, and introducing the amplification products carried by a cloning vector into cells, a plurality of unknown genes can be cloned simultaneously and conveniently, with a sequence corresponding to one of the employed primer linked at its 5'-end and the sequence from the vector DNA linked at its 3'-end. Repeated studies confirmed the reproducibility of the method and thus completed the present invention.

Therefore, the present invention provides a method of cloning DNA fragments comprising the steps of:
(a) cleaving a vector DNA of plasmid origin with a restriction enzyme,
(b) dephosphorylating an end of thus obtained cleaved DNA,
(c) separately obtaining a mixture of DNA fragments by cleaving a chromosomal DNA of a given organism with the same restriction enzyme as said restriction enzyme,
(d) obtaining a mixture of ligated DNAs through ligation using the dephosphorylated DNAs obtained in step (b) and the mixture of DNA fragments obtained in step (c),
(e) amplifying the ligated DNAs by PCR with arbitrary primers using the obtained mixture of ligated DNAs as templates, and
(f) introducing into a competent cell a cloning vector into which is incorporated a PCR product thus obtained.

According to the present method, a plurality of chromosomal DNA sequences of a given organism can be cloned simultaneously and very conveniently, with one of the nucleotide sequences corresponding to the arbitrary primers used in the PCR included at its 5'-end and the sequence from the vector DNA of plasmid origin, e.g., vector DNA of pUC origin, linked at its 3'-end via the restriction enzyme cleaved site. As unknown genes cloned by this method facilitate sequencing of the unknown genes, for they can be readily sequenced using the same primers, the method provides a very useful means in the search of genes for research and development of therapeutics of a variety of diseases.

In the above study, the genomic DNA of Streptococcus zooepidemicus was extracted and cloned according to the present invention using arbitrary primers. The DNA fragment set forth under SEQ ID NO:1 in the Sequence Listing was thus obtained, whose nucleotide sequence is similar to that of deoxyguanosine kinase/deoxyadenosine kinase subunit.

Therefore, the present invention provides a DNA having the nucleotide sequence set forth under SEQ ID NO:1 in the Sequence Listing and a protein having an amino acid sequence deduced therefrom which is set forth under SEQ ID NO:2.

In addition, a further cloning was performed with the extracted genomic DNA of Streptococcus zooepidemicus according to the present invention, but using different arbitrary primers, and gave a DNA fragment set forth under SEQ ID NO:3 in the Sequence Listing, whose nucleotide sequence is similar to that of hydroxymyristoyl-(acyl carrier protein) dehydratase and acetyl CoA carboxylase subunit.

Therefore, the present invention further provides the DNA having the nucleotide sequence set forth under SEQ ID NO:3 in the Sequence Listing, as well as proteins having respective amino acid sequences deduced therefrom which are set forth under SEQ ID NO:4 and NO:5.

Furthermore, the present invention provides expression vectors carrying one of the DNAs having aforementioned nucleotide sequences set forth under SEQ ID NO: 1 or 3 in the Sequence Listing.

Still further, the present invention provides host cells transformed with one of the aforementioned expression vectors.

In addition, the present invention provides antibodies directed to a protein having one of the aforementioned amino acid sequences set forth under SEQ ID NO:2, NO:4 or NO:5 in the Sequence Listing.

### DETAILED DESCRIPTION OF THE INVENTION

Chromosomal DNAs screened by the method of the present invention may be any chromosomal DNA, without being limited to eukaryotic or prokaryotic cells. Extraction of chromosomal DNAs may be performed by a method well known to those skilled in the art. Vectors of plasmid origin may be vectors of pUC origin, for example, among which pUC18 and other variety of vectors may be used. A restriction enzyme used for cleaving the vectors and chromosomal DNAs may be EcoRI or one of other variety of restriction enzymes. DNA fragments used as primers may be arbitrary ones and their length may be determined as desired. In general, the length of about 17 - 30 nucleotides is convenient. After incorporation into a cloning vector and then cloning into competent cells, DNA is extracted, and the presence of an insert can be detected by PCR performed using the same primers. After detected, the insert can be sequenced by a well known method.

The methods of producing plasmids and vectors that can express a desired gene including different variants obtained by the methods are well known to those skilled in the art: by inserting into an expression vector a DNA carrying a desired gene using a combination of restriction enzymes and ligase, a recombinant plasmid carrying the desired gene can be readily constructed. By introducing the thus obtained recombinant plasmid into different cells, the cells are transfected and thus transformed cells can be produced. Cells ranging from prokaryotic cells such as E. coli to eukaryotic cells such as yeast, insect, plant or animal cells may be utilized. In the present invention, the term "host cells" includes both prokaryotic and eukaryotic cells. [References: Vectors essential data. Gacesa P. and Ramji D.P., 166 pages. BIOS Scientific Publishers Limited 1994., John Wiley & Sons in association with BIOS Scientific Publisher Ltd. Expression vectors, pages 9-12.]

Introduction of a recombinant plasmid into host cells can be effected by calcium chloride method or electroporation. Calcium chloride method can provide efficient transformation without requiring any special apparatus. For higher efficiency, electroporation should be employed.
[References: Current protocols in molecular biology, 3 vols. Editied by Ausbel F.M. et al., John Wiley & Sons, Inc., Current Protocols, Vol. 1, unit 1.8: Introduction of plasmid DNA into cells, pages 1.8.1-1.8.10]

Two types of transfection are known which are generally carried out on animal cell lines, i.e. transient and permanent types. In transient transfection, transformed cells are cultured for 1 - 4 days to effect transcription and replication of the transfected gene, and the cells then are harvested and their DNA analyzed. Alternatively, in many studies, a stable transformant cell line is produced, in which the transfected gene is incorporated into a chromosome. For transfection, calcium phosphate method, electroporation, liposome fusion method, etc. are used.
[Reference: Current protocols in molecular biology, 3 vols. Edited by Ausubel F.M. et al., John Wiley & Sons, Inc., Current Protocols. vol. 1, chapter 9: Introduction of DNA into mammalian cells, pages 9.0.1-9.17.3.]

Polyclonal and monoclonal antibodies directed to the proteins (polypeptides) coded by the gene of the present invention, or to their fragments and analogues as well, are readily prepared using techniques well known in the art. Antibodies thus obtained may be useful as laboratory reagents and diagnostic agents for diseases associated with the gene of the present invention. The antibodies obtained may be widely used for preparation of antibody columns, in immunoprecipitation as well as for identification of antigen by Western blotting. In the present invention, the term "antibody" includes both monoclonal and polyclonal antibodies.

A general method for preparing a monoclonal antibody in mg-scale directed to the proteins coded by the gene of the present invention is as follows: mice are inoculated with the antigen protein to immunize, and the spleen is removed from those mice that exhibit sufficient antibody titers. Spleen cells are separated, and B cells selected are fused with myeloma cells of B cell origin to form hybridoma cells which secrete the antibody. The monoclonal antibody secreted from the hybridoma cells is purified from the culture medium by means of an affinity column, ion-exchange, or gel filtration, etc. Also, polyclonal antibody of the present invention can be prepared by a conventional method: using rabbits, horses, mice or guinea pigs as immunized animals, the antigen protein is inoculated along one of the schedules known to those skilled in the art to immunize the animals, and then IgG, etc. are isolated from their collected serum.
[Reference: Current protocols in molecular biology, 3 vols. Edited by Ausubel F.M. et al., John Wiley & Sons, Inc., Current Protocols, Vol. 2, chapter 11: Immunology, pages 11.0.1-11.16.13.]

The present invention is described in further detail with reference to an example below. It is not intended, however, that the scope of the present invention be restricted to the example.

### EXAMPLE

A genomic gene of Streptococcus zooepidemicus (S. zooepidemicus; a Lancefield group C streptococcus), which is a bacterium generally infective to certain animals such as horses, was extracted as follows: cultured bacteria (100 ml) was collected by centrifugation, and to this were added 5 ml of a buffer (10 mM Tris-HCl, 1 mM EDTA, pH8.0), 0.25 ml of 10 % SDS and 0.025 ml of 20 mg/ml proteinase, and allowed to react for 45 min at 37°C. Then, 0.948 ml of 5M NaCl was admixed, and 0.8 ml of 10 % hexadecyltrimethylammonium bromide dissolved in 0.7 M NaCl was further added, and reaction was allowed for 20 min at 65°C. The eluate thus obtained was treated with an equal volume of phenol/chloroform/isoamyl alcohol (25/24/1), and to this was further added isopropanol (0.6 volume) to precipitate DNA. The precipitated DNA was dried and then dissolved in a buffer (10 mM Tris-HCl, 1 mM EDTA, pH8.0) to make an appropriate volume. After decomposing residual RNA with RNase, the mixture was treated with an equal volume of phenol/chloroform/isoamyl alcohol (25/24/1), and to this was further added isopropanol (0.6 volume) to precipitate DNA. The precipitated DNA was dried and then dissolved in a buffer (10 mM Tris-HCl, 1 mM EDTA, pH8.0: TE) to make an appropriate volume.

The DNA thus obtained was subjected to restriction enzyme digestion as follows: about 1 µg of the DNA was treated with a restriction enzyme EcoRI (20 - 30 units), 5 µl of 10×reaction buffer (500 mM Tris-HCl (pH7.5), 100 mM MgCl₂, 10 mM dithiothreitol (DTT), 100 mM NaCl) and water (sterile distilled water) of an amount making a final volume of 50 µl, and allowed to react for 2 hrs at 37°C to cleave the DNA.

After the reaction, a 1/10 volume of 3M sodium acetate (pH5.4) and 2.5 volumes of cold ethanol (99.5%) were added to the reaction mixture and mixed, and the mixture thus obtained was allowed to stand in a freezer at -20°C overnight, then thawed and centrifuged to obtain precipitate, which was then washed again with 70% ethanol, dried, dissolved in 50 µl of water to adjust to a concentration of 1 µg/50 µl (ethanol precipitation).

Separately, 1 µg of pUC18 vector DNA was treated with a restriction enzyme EcoRI (20 - 30 units) and 2 µl of 10×reaction buffer (500 mM Tris-HCl (pH7.5), 100 mM MgCl₂, 10 mM dithiothreitol (DTT), 100 mM NaCl) and sterile distilled water of an amount making a final volume of 20 µl, and allowed to react for 2 hrs at 37°C to cleave the DNA.

To the thus obtained reaction mixture was added bovine intestine alkaline phosphatase (20 units) and reaction was allowed for 2 hrs at 37°C to dephosphorylate the DNA fragments. After the reaction, an equal volume of phenol saturated with the buffer solution was added, mixed and centrifuged (2 - 3 times). The aqueous phase was mixed with 1/10 volume of 3 M sodium acetate (pH5.4) and 2.5 volumes of cold ethanol (99.5%), the mixture solution was allowed to stand in a freezer at -20°C overnight, then thawed, and centrifuged to obtain precipitate, washed again with 70% ethanol, dried, and dissolved in 50 µl of sterile distilled water.

About 0.4 µg of the aforementioned genomic gene and 0.125 µg of the aforementioned pUC18 DNA, both cleaved with that restriction enzyme, were ligated with 1 µl of T4 DNA ligase (300 - 400 units), 5 µl of 10×reaction buffer (660 mM Tris-HCl (pH7.6), 66 mM MgCl₂, 100 mM DTT, 1 mM MATP) and sterile distilled water of an amount making a final volume of 50 µl, and allowed to react at 16°C overnight.

This ligation mixture solution was utilized. As arbitrary primers, primer RV (from Kokusai Shiyaku Kabusikikaisha), which has a nucleotide sequence set forth under SEQ ID NO:6 in the Sequence Listing, and primer mgaF417 (from Kokusai Shiyaku Kabusikikaisha), which has a nucleotide sequence set forth under SEQ ID NO:7 in the Sequence Listing, were chosen. PCR was performed using these materials: five µl of the above ligation mixture solution was admixed with 1 µl each of 20 pmol primers RV and mgaF417 (both prepared at 20 pmol/µl in a buffer containing 10 mM Tris-HCl, 1 mM EDTA, pH8.0 (TE)), 8 µl of 0.2 mM deoxyribonucleotide triphosphate mixture (composed of each 2 ml of four different dNTPs), 0.5 µl of Taq polymerase (0.5 unit), 10 µl of 10×reaction buffer [100 mM Tris-HCl (pH8.3), 500 mM KCl, 15 mM MgCl₂, 0.01% (W/V) gelatin] and 6 µl of 25 mM MgCl₂, and then sterile distilled water was added to make a final volume of 100 µl, and this was followed by the addition of 2 drops of mineral oil. The conditions for PCR cycles were as follows: thirty cycles of denaturation (94°C, 30 sec), annealing (55°C, 2 min) and extension (78°C, 2 min).

The PCR products thus obtained were ligated to a commercially available TA cloning vector in the following manner: five µl of sterile distilled water was admixed with 1 µl of 10 × ligation buffer [100 mM Tris-HCl (pH8.3), 500 mM KCl,25 mM MgCl₂, 0.01% (W/V) gelatin], 1 µl of TA cloning vector [pCR2.1; INVITROGEN: 25 ng/ml in a buffer containing 10 mM Tris-HCl, 1 mM EDTA (pH8)], 1 µl of the above PCR products and 1 µl of T4 DNA ligase, and the mixture was allowed to react at 14°C for 4 hours or longer.

Using the thus obtained ligation reaction mixture, introduction into competent cells, cloning, and selection of transformed cells were performed: to 50 µl of TOP10F⁷ (INVITROGEN) competent cells (1×10⁸ cells/ml)were added 2 µl of 0.5 M 2-ME (2-mercaptoethanol), and, after mixing, 2 µl of the ligation reaction solution was added, and the mixture was set on ice for 30 min. Then, reaction was allowed at 42°C for 30 sec, and the reaction mixture was set on ice for 2 min. To this reaction mixture was added 250 µl of SOC medium (2% Tryptone, 0.5% yeast extract, 10 mM NaCl, 2.5 mM KCl, 10 mM MgCl₂, 10 mM MgSO₄, 20 mM glucose), and the mixture was allowed to react at 37°C for 1 hour. Fifty to a hundred µl of the reaction mixture was plated over a LB plate (1.0 Tryptone, 0.5% yeast extract, 1.0% NaCl) containing 40 µl of X-gal (40 mg/ml) and 40 µl of IPTG (23.8 mg/ml) and kanamycin (50 µg/ml). After allowing to react overnight at 37 °C , white colonies were selected.

PCR was carried out using the same primers (RV and mgaF4 17) for detection of inserts. As a result, 31 clones were found. Ten clones of different sizes were chosen and sequenced. As a result, inserts were classified into two groups according to their nucleotide sequences at 5'- and 3'-ends: a first group of clones (8 clones) has the same sequence as RV at its 5'-end and has a fragment originating from pUC18 (including a EcoRI cleaving site) at its 3'-end, and a second group of clones (2 clones) has the same sequence as mgaF417 at its 5'-end and has a fragment originating from pUC (including a EcoRI cleaving site) at its 3'-end.

Out of the aforementioned clones, set forth under SEQ ID NO: 1 in the Sequence Listing is a partial nucleotide sequence originating from the chromosomal DNA of Streptococcus zooepidemicus included in a representative one of the clones which have the same sequence as RV at their 5'-end and a fragment (including an EcoRI cleaving site) originating from pUC18 at their 3'-end. To the 5'-end of the nucleotide sequence is attached the same nucleotide sequence as that of primer RV. The guanine (g) at the 3'-end is the guanine (g) located at the 5'-end of the recognition sequence "gaattc" for the restriction enzyme EcoRI. With a sequence originating from pUC18 linked downstream to the guanine via the rest of the sequence, "aattc", the 3'-end of the sequence set forth under SEQ ID NO: 1 and the connection region of the sequence from pUC18 make up a EcoRI cleaving site.

Under SEQ ID NO:2 in the Sequence Listing is set forth a partial amino acid sequence deduced from the partial nucleotide sequence of the chromosomal DNA of Streptococcus zooepidemicus. Homology search using BLAST (Basic Local Alignment Search Tool) software for the partial amino acid sequence showed that the sequence is identical in 115 out of the 205 residues (56%) with that of deoxyguanosine kinase subunit 2 (deoxyguanosine kinase)(Lactobacillus acidophilus) and in 104 out of the 199 residues (52%) with that of deoxyguanosine kinase/deoxyadenosine kinase subunit 1 (deoxyadenosine kinase)(Lactobacillus acidophilus). Moreover, it was revealed that the sequence contains a glycine-rich sequence (IGAGKSSL) characteristic of ATP binding sits, a DRF motive which is associated with a nucleic acid binding site, and an arginine-rich site (RIEKRGRR) which is thought to be involved in the binding of the phosphate group of ATP. According to these findings, this gene fragment from S. zooepidemicus is thought to be a fragment of a gene similar to deoxyguanosine kinase/deoxyadenosine kinase. Nucleic acid analogues so far used as chemotherapeutics for leukemia are known to inhibit DNA synthesis after phosphorylated by intracellular deoxyribonucleoside kinase. Recently, Chaoyong, Zhu et al. (1998, J. Biol. Chem., vol. 273, pp.14707-14711) reported that the cytotoxicity to cancer cells of certain nucleic acid analogues [2-chloro-2'-deoxyadenosine (CdA), 9-β-D-arabinofranosylguanine (araG), and 2',2'-difluorodeoxyguanosine (dFdG)] are enhanced by over expression of mitochondrial deoxyguanosine kinase within cancer cells. In light of these findings, the above obtained gene of a deoxyguanosine kinase/deoxyadenosine kinase analogue is expected to have similar functions.

Out of the aforementioned clones, set forth under SEQ ID NO:3 in the Sequence Listing is a partial nucleotide sequence originating from the chromosomal DNA of Streptococcus zooepidemicus included in a representative one of the clones which have the same sequence as mgaF417 at their 5'-end and a fragment (including an EcoRI cleaving site) originating from pUC18 at their 3'-end. To the 5'-end of the partial nucleotide sequence is attached the same nucleotide sequence as that of primer mgaF417. The sequence "gaattc" at the 3'-end is the recognition sequence by the restriction enzyme EcoRI. With a sequence originating from pUC18 linked downstream via the recognition sequence, the 3'-end region of the sequence set forth under SEQ ID NO:3 and the connection region of the sequence from pUC18 make up a EcoRI cleaving site.

Under SEQ ID NO:4 and SEQ ID NO:5 in the Sequence Listing are set forth two partial amino acid sequences deduced from the respective partial nucleotide sequences of the chromosome of Streptococcus zooepidemicus. Homology search using BLAST (Basic Local Alignment Search Tool) software for these partial sequences showed that the amino acid sequence set forth under SEQ ID NO:4 is identical in 72 out of 121 residues (59%) with that of hydroxymyristoyl- (acyl carrier protein) dehydratase (Bacillus subtilis) and that the amino acid sequence set forth under SEQ ID NO:5 is identical in 45 out of 63 residues at its N-terminus (71%) with that of acetyl-CoA carboxylase subunit (biotin carboxylase subunit). These results indicate that the gene fragment originating from S. zooepidemicus set forth under SEQ ID NO:3 is a fragment of a gene similar to hydroxymyristoyl-(acyl carrier protein) dehydratase and acetyl CoA carboxylase subunit (biotin carboxylase subunit).

As the present invention makes it possible to conveniently screen fragments of new genes, it enables easier and quicker search than before of genes important for the development of therapeutics.

## Claims

1. A method of cloning DNA fragments comprising the steps of:
(a) cleaving a vector DNA of plasmid origin with a restriction enzyme,
(b) dephosphorylating an end of thus obtained cleaved DNA,
(c) separately obtaining a mixture of DNA fragments by cleaving a chromosomal DNA of a given organism with the same restriction enzyme as said restriction enzyme,
(d) obtaining a mixture of ligated DNAs through ligation using the dephosphorylated DNAs obtained in step (b) and the mixture of DNA fragments obtained in step (c),
(e) amplifying the ligated DNAs by PCR with arbitrary primers using the obtained mixture of ligated DNAs as templates, and
(f) introducing into a competent cell a cloning vector into which is incorporated a PCR product thus obtained.

2. A DNA having a nucleotide sequence set forth under SEQ ID NO:1 in the Sequence Listing, which is obtainable from the chromosomal DNA of Streptococcus zooepidemicus according to the method of claim 1.

3. A DNA having a nucleotide sequence set forth under SEQ ID NO:3 in the Sequence Listing, which is obtainable from the chromosomal DNA of Streptococcus zooepidemicus according to the method of claim 1.

4. A protein having an amino acid sequence set forth under SEQ ID NO:2 in the Sequence Listing.

5. A protein having an amino acid sequence set forth under SEQ ID NO:4 in the Sequence Listing.

6. A protein having an amino acid sequence set forth under SEQ ID NO:5 in the Sequence Listing.

7. An expression vector carrying a DNA of claim 2.

8. An expression vector carrying a DNA of claim 3.

9. A host cell transformed with the expression vector of claim 7.

10. A host cell transformed with the expression vector of claim 8.

11. An antibody directed to a protein of one of claims 4, 5 and 6.


SEQUENCE LISTING

<110> JCR Pharmaceuticals Co., Ltd.

<120> A cloning method for DNA fragments using arbitrarily primed PCR

<130> P54-99

<160> 7

<210> 1
<211> 627
<212> DNA
<213> Streptococcus zooepidemicus

<220>
<221> CDS
<222> (2)...(616)

<400> 1
a atc ggc gca ggc aag agt tcc ctt gct gct gca ctg ggt gag cat tta 49
Ile Gly Ala Gly Lys Ser Ser Leu Ala Ala Ala Leu Gly Glu His Leu
1               5                   10                  15

gga aca gag gta ttt tac gag gct gtt gat aac aat cct gtt ctt gat 97
Gly Thr Glu Val Phe Tyr Glu Ala Val Asp Asn Asn Pro Val Leu Asp
            20                  25                  30

ctg tat tac caa gac cct aaa aaa tat gcc ttt tta ttg caa att ttc 145
Leu Tyr Tyr Gln Asp Pro Lys Lys Tyr Ala Phe Leu Leu Gln Ile Phe
        35                  40                  45

ttt ttg aat aag cgc ttc aaa tct att aaa gag cct atc cag gca gac 193
Phe Leu Asn Lys Arg Phe Lys Ser Ile Lys Glu Pro Ile Gln Ala Asp

    50                  55                  60

aat aat att ctt gac cgc tca atc ttt gaa gat gag ctc ttc ttg aca 241
Asn Asn Ile Leu Asp Arg Ser Ile Phe Glu Asp Glu Leu Phe Leu Thr
65                  70                  75                  80

ctt aac tat aaa aat gga aat gtt acc aag aca gat ctt gaa att tac 289
Leu Asn Tyr Lys Asn Gly Asn Val Thr Lys Thr Asp Leu Glu Ile Tyr
                85                  90                  95

caa gag ctc tta gcc aat atg cta gag gag ctt gag gga atg cct aaa 337
Gln Glu Leu Leu Ala Asn Met Leu Glu Glu Leu Glu Gly Met Pro Lys
            100                 105                 110

aaa cgt cct gac ctg ctg att tat att gat gtc tcc ttt gag aag atg 385
Lys Arg Pro Asp Leu Leu Ile Tyr Ile Asp Val Ser Phe Glu Lys Met
        115                 120                 125

cta gag cgc att gaa aag cgt ggc agg cgg ttc gag cag gtt gat gac 433
Leu Glu Arg Ile Glu Lys Arg Gly Arg Arg Phe Glu Gln Val Asp Asp
    130                 135                 140

aat cct gac cta gag gcc tat tac cat cag gta cat ggc gaa tac cca 481
Asn Pro Asp Leu Glu Ala Tyr Tyr His Gln Val His Gly Glu Tyr Pro
145                 150                 155                 160

acc tgg tac gag cgt tat gac gtc tca cct aag atg agg att gat gga 529
Thr Trp Tyr Glu Arg Tyr Asp Val Ser Pro Lys Met Arg Ile Asp Gly
                165                 170                 175

aac aag ctt gat ttt gtg caa aac cca gag gat ctg gca acc gtc ctg 577
Asn Lys Leu Asp Phe Val Gln Asn Pro Glu Asp Leu Ala Thr Val Leu
            180                 185                 190

caa atg att gat gaa aag cta aaa acc tta gat tta ctg taaaaacaag g    627
Gln Met Ile Asp Glu Lys Leu Lys Thr Leu Asp Leu Leu
        195                 200                 205

<210> 2
<211> 205
<212> PRT
<213> Streptococcus zooepidemicus

<400> 2
Ile Gly Ala Gly Lys Ser Ser Leu Ala Ala Ala Leu Gly Glu His Leu
1               5                   10                  15

Gly Thr Glu Val Phe Tyr Glu Ala Val Asp Asn Asn Pro Val Leu Asp
            20                  25                  30

Leu Tyr Tyr Gln Asp Pro Lys Lys Tyr Ala Phe Leu Leu Gln Ile Phe
        35                  40                  45

Phe Leu Asn Lys Arg Phe Lys Ser Ile Lys Glu Pro Ile Gln Ala Asp
    50                  55                  60

Asn Asn Ile Leu Asp Arg Ser Ile Phe Glu Asp Glu Leu Phe Leu Thr
65                  70                  75                  80

Leu Asn Tyr Lys Asn Gly Asn Val Thr Lys Thr Asp Leu Glu Ile Tyr
                85                  90                  95

Gln Glu Leu Leu Ala Asn Met Leu Glu Glu Leu Glu Gly Met Pro Lys
            100                 105                 110

Lys Arg Pro Asp Leu Leu Ile Tyr Ile Asp Val Ser Phe Glu Lys Met
        115                 120                 125

Leu Glu Arg Ile Glu Lys Arg Gly Arg Arg Phe Glu Gln Val Asp Asp
    130                 135                 140

Asn Pro Asp Leu Glu Ala Tyr Tyr His Gln Val His Gly Glu Tyr Pro
145                 150                 155                 160

Thr Trp Tyr Glu Arg Tyr Asp Val Ser Pro Lys Met Arg Ile Asp Gly
                165                 170                 175

Asn Lys Leu Asp Phe Val Gln Asn Pro Glu Asp Leu Ala Thr Val Leu
            180                 185                 190

Gln Met Ile Asp Glu Lys Leu Lys Thr Leu Asp Leu Leu
        195                 200                 205

<210> 3
<211> 630
<212> DNA
<213> Streptococcus zooepidemicus

<220>
<221> CDS
<222> (1)...(402)
<220>
<221> CDS
<222> (437)...(628)

<400> 3
caa gaa gca ctg cca cat cgt tgc cca atg ctg ctt gtt gat agg att      48
Gln Glu Ala Leu Pro His Arg Cys Pro Met Leu Leu Val Asp Arg Ile
  1               5                  10                  15

tta gag gct tca gac gat gaa att gtt gcc atc aaa aat gtc act atc      96
Leu Glu Ala Ser Asp Asp Glu Ile Val Ala Ile Lys Asn Val Thr Ile

            20                  25                  30

aat gag ccc ttc ttt aac ggt cat ttt cct cag tat cca gtc atg cca 144
Asn Glu Pro Phe Phe Asn Gly His Phe Pro Gln Tyr Pro Val Met Pro
        35                  40                  45

ggt gtt ttg atc atg gag gcc ttg gca caa act gct ggc gtc ttg gag 192
Gly Val Leu Ile Met Glu Ala Leu Ala Gln Thr Ala Gly Val Leu Glu
    50                  55                  60

cta tca aaa gag gaa aat aaa ggc aag ctt gtt ttt tac gct ggt atg 240
Leu Ser Lys Glu Glu Asn Lys Gly Lys Leu Val Phe Tyr Ala Gly Met
65                  70                  75                  80

gac aag gta gaa ttt aaa aag cag gtg gtt ccg gga gac cag cta gtc 288
Asp Lys Val Glu Phe Lys Lys Gln Val Val Pro Gly Asp Gln Leu Val
                85                  90                  95

atg aca gct agg ttt att aag cgt cgt ggg aca ata gca gtt gtt gag 336
Met Thr Ala Arg Phe Ile Lys Arg Arg Gly Thr Ile Ala Val Val Glu
            100                 105                 110

gcc aag gca gag gtt gat ggc aaa tta cca gct agt ggg acc ttg act 384
Ala Lys Ala Glu Val Asp Gly Lys Leu Pro Ala Ser Gly Thr Leu Thr
        115                 120                 125

ttt gct ttt ggg cag taa aagactaatc gtctgtggag gaaaaaagaa acct atg 439
Phe Ala Phe Gly Gln Met
130 1

ttt aac aaa atc tta att ccc aat cgt ggt gaa ata tca gtg cgg att 487
Phe Asn Lys Ile Leu Ile Pro Asn Arg Gly Glu Ile Ser Val Arg Ile
5 10 15

att cgt gca gca cga gaa tta ggc att tcc aca gtt gct gtt tat tcc     535
Ile Arg Ala Ala Arg Glu Leu Gly Ile Ser Thr Val Ala Val Tyr Ser
        20                  25                  30

gag gcc gat aaa gag gct tta cat acg atc ttg gca gac cag gcc atc     583
Glu Ala Asp Lys Glu Ala Leu His Thr Ile Leu Ala Asp Gln Ala Ile
    35                  40                  45

tgt att gga ccg tca aga tca aag gaa tcc tat ctc cat atg aat tc      630
Cys Ile Gly Pro Ser Arg Ser Lys Glu Ser Tyr Leu His Met Asn
50                  55                  60

<210> 4
<211> 133
<212> PRT
<213> Streptococcus zooepidemicus

<400> 4
Gln Glu Ala Leu Pro His Arg Cys Pro Met Leu Leu Val Asp Arg Ile
1               5                   10                  15

Leu Glu Ala Ser Asp Asp Glu Ile Val Ala Ile Lys Asn Val Thr Ile
            20                  25                  30

Asn Glu Pro Phe Phe Asn Gly His Phe Pro Gln Tyr Pro Val Met Pro
        35                  40                  45

Gly Val Leu Ile Met Glu Ala Leu Ala Gln Thr Ala Gly Val Leu Glu
    50                  55                  60

Leu Ser Lys Glu Glu Asn Lys Gly Lys Leu Val Phe Tyr Ala Gly Met
65                  70                  75                  80

Asp Lys Val Glu Phe Lys Lys Gln Val Val Pro Gly Asp Gln Leu Val

                85                  90                  95

Met Thr Ala Arg Phe Ile Lys Arg Arg Gly Thr Ile Ala Val Val Glu
            100                 105                 110

Ala Lys Ala Glu Val Asp Gly Lys Leu Pro Ala Ser Gly Thr Leu Thr
        115                 120                 125

Phe Ala Phe Gly Gln
    130

<210> 5
<211> 64
<212> PRT
<213> Streptococcus zooepidemicus

<400> 5
Met Phe Asn Lys Ile Leu Ile Pro Asn Arg Gly Glu Ile Ser Val Arg
1               5                   10                  15

Ile Ile Arg Ala Ala Arg Glu Leu Gly Ile Ser Thr Val Ala Val Tyr
            20                  25                  30

Ser Glu Ala Asp Lys Glu Ala Leu His Thr Ile Leu Ala Asp Gln Ala
        35                  40                  45

Ile Cys Ile Gly Pro Ser Arg Ser Lys Glu Ser Tyr Leu His Met Asn
    50                  55                  60

<210> 6
<211> 17
<212> DNA
<213> Bacteriophage M13

<400> 6
caggaaacag ctatgac                                                    17

<210> 7
<211> 18
<212> DNA
<213> Streptococcus pyogenes

<400> 7
ggagatgaac accagatt                                                   18